# EUROPEAN PATENT SPECIFICATION

(11) **EP 3 918 332 B1**
(45) Date of publication and mention of the grant of the patent: **19.03.2025**
(21) Application number: 20701777.3
(22) Date of filing: 29.01.2020
(51) Int. Cl.: G01N 33/574

(54) **METHODS AND COMPOSITIONS FOR IDENTIFYING WHETHER A SUBJECT SUFFERING FROM A CANCER WILL ACHIEVE A RESPONSE WITH AN IMMUNE-CHECKPOINT INHIBITOR**
VERFAHREN UND ZUSAMMENSETZUNGEN ZUR BESTIMMUNG, OB EINE AN KREBS LEIDENDE PERSON EINE REAKTION MIT EINEM IMMUN-CHECKPOINT-INHIBITOR ERREICHT
PROCÉDÉS ET COMPOSITIONS PERMETTANT D'IDENTIFIER SI UN SUJET SOUFFRANT D'UN CANCER VA ÊTRE RÉACTIF À UN INHIBITEUR DE POINT DE CONTRÔLE IMMUNITAIRE

(30) Priority: 30.01.2019 EP 19305114
(43) Date of publication of application: 08.12.2021
(73) Proprietor: INSERM (Institut National de la Santé et de la Recherche Médicale), 75013 Paris (FR); Université Paris Cité, 75006 Paris (FR); Assistance Publique-Hôpitaux de Paris (APHP), 75004 Paris (FR)
(72) Inventor: TARTOUR, Eric, 75015 Paris (FR); BEN HAMOUDA, Nadine, 75015 Paris (FR); OUDARD, Stéphane, 75015 Paris (FR); EPAILLARD, Nicolas, 75015 Paris (FR)
(74) Representative: Inserm Transfert
(86) International application number: PCT/EP2020/052165
(87) International publication number: WO 2020/157131

(56) References cited:
- MELANIE RUF ET AL: "Interaction of tumor cells with infiltrating lymphocytes via CD70 and CD27 in clear cell renal cell carcinoma", ONCOIMMUNOLOGY, vol. 4, no. 12, 29 May 2015 (2015-05-29), pages e1049805, XP055602971, DOI: 10.1080/2162402X.2015.1049805
- RACHEL E. O'NEILL ET AL: "T Cell-Derived CD70 Delivers an Immune Checkpoint Function in Inflammatory T Cell Responses", THE JOURNAL OF IMMUNOLOGY, vol. 199, no. 10, 18 October 2017 (2017-10-18), US, pages 3700 - 3710, XP055602983, ISSN: 0022-1767, DOI: 10.4049/jimmunol.1700380
- M. J KERSTEN ET AL: "Elevation of cerebrospinal fluid soluble CD27 levels in patients with meningeal localization of lymphoid malignancies", BLOOD, 1 January 1996 (1996-01-01), United States, pages 1985 - 1989, XP055602940, Retrieved from the Internet <URL:http://www.bloodjournal.org/content/bloodjournal/87/5/1985.full.pdf>
- ALLEN W HO ET AL: "CD27-CD70 interactions in the pathogenesis of Waldenström macroglobulinemia", BLOOD JOURNAL, vol. 112, no. 12, 1 December 2008 (2008-12-01), pages 4683 - 4689, XP055602935, DOI: 10.1182/blood-2007-04-
- RENEEN DONAHUE ET AL: "Analyses of the peripheral immunome following multiple administrations of avelumab, a human IgG1 anti-PD-L1 monoclonal antibody", JOURNAL FOR IMMUNOTHERAPY OF CANCER, BIOMED CENTRAL LTD, LONDON, UK, vol. 5, no. 1, 21 February 2017 (2017-02-21), pages 1 - 16, XP021242481, DOI: 10.1186/S40425-017-0220-Y

## Description

### FIELD:

The disclosure is in the field of oncology. More particularly, the disclosure relates to methods and composition for determining whether a subject suffering from kidney cancer will achieve a response with an anti-PD-1, anti-PD-L1 or anti-PD-L2 antibody treatment.

### BACKGROUND:

The anti-PD-1 antibody (nivolumab) has been approved in second-line in metastatic renal cell carcinoma tumors following the Phase 3 study showing the superiority of this treatment compared to anti-angiogenic therapy (everolimus) [1]. 25% of patients respond to this treatment with a 5.5 months survival advantage compared to the standard treatment.

To date, conventional biomarkers such as PD-L1 or mutational load or infiltration by intratumoral CD8+T cells do not predict the response to this immunotherapy treatment in patients with metastatic renal cancer [1-3].

In a previous study of our team, we showed that co-expression of PD-1 and Tim-3 on CD8+T cells was associated with a poor prognosis. Functional studies showed that these CD8+T cells were not functional [4]. In line with our results, other preliminary studies have shown that this co-expression can predict the response to immunotherapy [5, 6]. Thus, in a recent clinical study, targeting the PD-1 / PD-L1 axis (Program Death Ligand 1), it has been shown that CD8+T cells expressing PD-1 and CD28 are required for the efficacy of treatment. Interestingly, CD8+T cells co-expressing PD-1 and Tim-3 are predominantly CD28 negative [7].

The use of immunotherapy more particularly the use of immune checkpoint inhibitors for cancers has become widespread in recent decades and is used to treat both solid and hematological malignances. However, only some patients respond to the immune checkpoint inhibitors treatment. Accordingly, identifying new biomarkers will be needed to identify whether a subject will respond to the treatment with an immune checkpoint inhibitor.

### SUMMARY OF THE INVENTION:

The invention relates to a method for determining whether a subject suffering from kidney cancer will achieve a response with an anti-PD-1, anti-PD-L1 or anti-PD-L2 antibody comprising the steps of i) quantifying the level of soluble CD27 (sCD27) in a biological sample obtained from the subject treated with an anti-PD-1, anti-PD-L1 or anti-PD-L2 antibody, ii) comparing the level of soluble CD27 quantified at step i) with its corresponding predetermined reference value and iii) concluding that the subject will not respond to the treatment when the level of soluble CD27 is higher than its corresponding predetermined reference value or concluding that the subject will respond to the treatment when the level of soluble CD27 is lower than its corresponding predetermined reference value.

The invention also refers to an anti-PD-1, anti-PD-L1 or anti-PD-L2 antibody for use in the treatment of kidney cancer in a subject identified as a responder to an anti-PD-1, anti-PD-L1 or anti-PD-L2 antibody treatment, comprising the steps of i) quantifying the level of soluble CD27 in a biological sample obtained from the subject before being treated with an anti-PD-1, anti-PD-L1 or anti-PD-L2 antibody; ii) providing an evaluation based on the quantity of the soluble CD27 in the biological sample of said subject; and iii) treating said subject with the anti-PD-1, anti-PD-L1 or anti-PD-L2 antibody if said subject is identified as responder to said treatment at step ii).

The invention also refers to use of a kit or device for performing the method for determining whether a subject suffering from kidney cancer will achieve a response with an anti-PD-1, anti-PD-L1 or anti-PD-L2 antibody according to the invention, wherein the kit comprises means for determining the level of the soluble CD27 in a biological sample.

In particular, the invention is defined by claims.

### DETAILED DESCRIPTION:

Inventors have worked with two cohorts of patients: 1) A colcheckpoint cohort set up at the Georges Pompidou European Hospital (CPP: 2015-08-04-MS2) including since 2016 all the patients treated by immunotherapy (Anti-PD-1 / PD-L1) since 2016 and for whom blood and tissue samples are routinely performed after collection of patient consent. 27 renal cell cancer patients derived from this cohort were included in this study. 22 of these patients had clear cell kidney cancer. 2) The other set of specimens came from the Preinsut clinical study, where renal cell cancer patients received two cycles of sunitinib before nephrectomy [8]. 27 patients derived from this cohort were also included in this study.

Inventors have identified a soluble marker, CD27, present in the plasma of patients with renal cell cancer whose pre-treatment concentrations predict the response to anti-PD-1 / PD-L1. This marker appears more as a predictive marker of response to anti-PD1 / PD-L1 treatment, than as a prognostic marker. Indeed, it is not associated with better survival in patients with metastatic renal cell cancer treated with an antiangiogenic agent. This marker is not correlated with conventional clinical markers of severity that classify patients with metastatic renal cancer.

### Method for predicting a response to an immune checkpoint inhibitor treatment

Accordingly, in a first aspect, the invention relates to a method for determining whether a subject suffering from kidney cancer will achieve a response with an immune-checkpoint inhibitor selected from the group consisting of anti-PD-1, anti-PD-L1 or anti-PD-L2 antibody comprising the steps of i) quantifying the level of soluble CD27 in a biological sample obtained from the subject treated with an anti-PD-1, anti-PD-L1 or anti-PD-L2 antibody, ii) comparing the level of soluble CD27 quantified at step i) with its corresponding predetermined reference values and iii) concluding that the subject will not respond to the treatment when the level of soluble CD27 is higher than its corresponding predetermined reference value or concluding that the subject will respond to the treatment when the level of soluble CD27 is lower than its corresponding predetermined reference value.

As used herein, the terms "will achieve a response" or "respond" refer to the response to a treatment of the subject suffering from kidney cancer. Typically such treatment induces, ameliorates or otherwise causes an improvement in the pathological symptoms, disease progression or physiological conditions associated with or resistance to succumbing to a cancer. In particular, in the context of the invention, the term "respond" refers to the ability of an anti-PD-1, anti-PD-L1 or anti-PD-L2 antibody to an improvement of the pathological symptoms, thus, the subject presents a clinical improvement compared to the subject who does not receive the treatment. The said subject is considered as a "responder" to the treatment. The term "not respond" refers to a subject who does not present any clinical improvement to the treatment with an anti-PD-1, anti-PD-L1 or anti-PD-L2 antibody treatment. This subject is considered as a "non-responder" to the treatment. Accordingly, the subject as considered "non-responder" has a particular monitoring in the therapeutic regimen. In a particular embodiment, the response to a treatment is determined by Response evaluation criteria in solid tumors (RECIST) criteria. This criteria refers to a set of published rules that define when tumors in cancer subjects improve ("respond"), stay the same ("stabilize"), or worsen ("progress") during treatment. In the context of the invention, when the subject is identified as responder, it means that said subject improves overall and progression-free survival (OS/PFS). More particularly, the soluble CD27 is a tool to determine the overall survival (OS) of the subject before starting a treatment with an immune checkpoint inhibitor.

As used herein, the term "Overall survival (OS)" denotes the percentage of subject in a study or treatment group who are still alive for a certain period of time after they were diagnosed with or started treatment for a disease, such as a cancer (according to the invention).

As used herein, the term "cancer" refers to a malignant growth or tumour resulting from an uncontrolled division of cells.

As used herein, the terms "kidney cancer," "renal cancer," or "renal cell carcinoma" refer to cancer that has arisen from the kidney. The terms "renal cell cancer" or "renal cell carcinoma" (RCC), as used herein, refer to cancer which originates in the lining of the proximal convoluted tubule. More specifically, RCC encompasses several relatively common histologic subtypes: clear cell renal cell carcinoma, papillary (chromophil), chromophobe, collecting duct carcinoma, and medullary carcinoma. Clear cell renal cell carcinoma (ccRCC) is the most common subtype of RCC. In a particular embodiment, the cancer is a metastatic renal cell carcinoma.

As used herein, the term "subject" denotes a mammal, such as a rodent, a feline, a canine, and a primate. Particularly, the subject according to the invention is a human. More particularly, the subject according to the invention has or is susceptible to have renal cell cancer (RCC). In particular embodiment, the subject has or is susceptible to have lung cancer.

As used herein, the term "biological sample" refers to any sample obtained from a subject, such as a serum sample, a plasma sample, a urine sample, a blood sample, a lymph sample, or a tissue biopsy. In a particular embodiment, biological sample for the determination of an expression level includes samples such as a blood sample, a lymph sample, or a biopsy. In a particular embodiment, the biological sample is a blood sample. In another embodiment, the biological sample is a plasma sample.

As used herein, the term "CD27" is a member of the tumor necrosis factor receptor superfamily. It is currently of interest to immunologists as a co-stimulatory immune checkpoint molecule. CD27 binds to ligand CD70, and plays a key role in regulating B-cell activation and immunoglobulin synthesis. A soluble form of CD27 (sCD27), a 32-kD protein identical to the extracellular domain of membrane-bound CD27, can be released after lymphocyte activation by differential splicing of the receptor protein or shedding from the cell surface by proteases.

As used herein, the term "level of soluble CD27" refers to the concentration of soluble CD27. Typically, the level or concentration of the soluble CD27 gene may be determined by any technology known by a person skilled in the art. In particular, the concentration may be measured at the genomic and/or nucleic and/or protein level. In a particular embodiment, the expression level of gene is determined by measuring the amount of nucleic acid transcripts of each gene. In another embodiment, the expression level is determined by measuring the amount of each gene corresponding protein. The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-known in the art. From the mRNA or cDNA sample, the amount of nucleic acid transcripts may be measured using any technology known by a man skilled in the art, including nucleic microarrays, quantitative PCR, microfluidic cards, and hybridization with a labelled probe. In a particular embodiment, the expression level is determined by using quantitative PCR. Quantitative, or real-time, PCR is a well-known and easily available technology for those skilled in the art and does not need a precise description. Methods for determining the quantity of mRNA are well known in the art. For example the nucleic acid contained in the biological sample is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous. Other methods of amplification include ligase chain reaction (LCR), transcription-mediated amplification (TMA), strand displacement amplification (SDA) and nucleic acid sequence based amplification (NASBA). Nucleic acids having at least 10 nucleotides and exhibiting sequence complementarity or homology to the mRNA of interest herein find utility as hybridization probes or amplification primers. It is understood that such nucleic acids do not need to be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin). Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate). The nucleic acid primers or probes used in the above amplification and detection method may be assembled as a kit. Such a kit includes consensus primers and molecular probes. A kit also includes the components necessary to determine if amplification has occurred. The kit may also include, for example, PCR buffers and enzymes; positive control sequences, reaction control primers; and instructions for amplifying and detecting the specific sequences. In a particular embodiment, the method of the invention comprises the steps of providing total RNAs extracted from a biological sample and subjecting the RNAs to amplification and hybridization to specific probes, more particularly by means of a quantitative or semi-quantitative RT-PCR. In another embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a biological sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

In a particular embodiment, the level of soluble CD27 (sCD27) present in a plasma sample obtained from a subject is determined for example by detecting the amount of sCD27 present in the plasma sample. In some embodiments, to determine the amount of sCD27, the sample is contacted with a specific binding agent (such as an antibody, for example a monoclonal antibody anti-CD27 or a protein, such as CD70) that specifically binds sCD27 and the amount of the specific binding agent bound to sCD27 is detected. In the context of the invention, the Procartaplex kit (Thermofischer) was used to carry out a panel of soluble inhibitor or activator receptor (BTLA, GITR, HVEM, IDO, LAG-3, PD-1, PD-L1, PD-L2, Tim-3, CD28, CD80, 4-1BB, CD27, and CTLA-4).

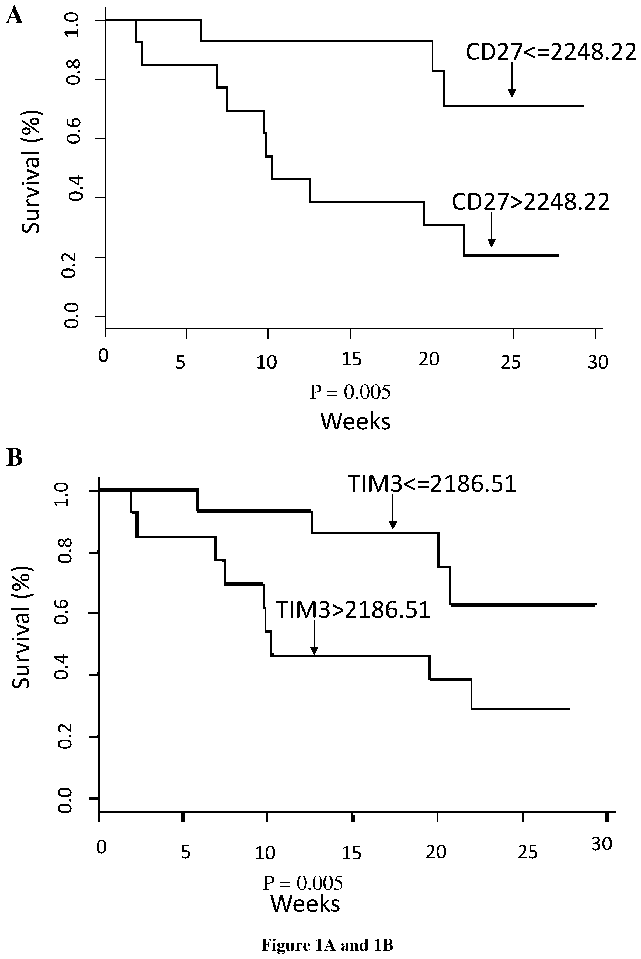

In some embodiments, the amount of sCD27 present in the plasma sample is detected by mass spectrometry. In some embodiments, the amount of sCD27 present in the serum sample is detected by measuring the activity of sCD27 in the plasma sample. This amount is compared with a control value. Typically, Log Rank (Kaplan Meier) or Cox tests were used to correlate the different parameters with patient survival. The different variables were analyzed either qualitatively with a threshold at the median for dichotomizing them or quantitatively (Cox model). Patients were divided into two groups selected depending on their plasma CD27 concentrations (above or below the median). Patient survival was determined from the start of treatment. A Log Rank test was performed to compare the survival of the 2 groups of patients. For the CD27 marker, this correlation was found whatever the statistical test used : Log Rank (qualitative variable) (p = 0.005) (Fig 1A), or Cox model (Quantitative variable) (Table 1) (p = 0.04).

In some embodiments, a score which is a composite of the level of the soluble CD27 is determined and compared to a reference value. When a high concentration of soluble CD27 than the reference value is determined, it is indicative that the subject will not achieve a response to an immune checkpoint inhibitor. When a low concentration of soluble CD27 than the reference value is determined, it is indicative that the subject will respond to an immune checkpoint inhibitor. Typically, the predetermined reference value is a threshold value or a cut-off value, which can be determined experimentally, empirically, or theoretically. A threshold value can also be arbitrarily selected based upon the existing experimental and/or clinical conditions, as would be recognized by a person of ordinary skilled in the art. For example, retrospective measurement of the level of the soluble CD27 in properly banked historical plasma samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the expression level of the soluble CD27 in a group of reference, one can use algorithmic analysis for the statistic treatment of the expression levels determined in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

In a particular embodiment, the method according to the invention further comprises a step of classification of subject by an algorithm and determining whether a subject will achieve a response to an anti-PD-1, anti-PD-L1 or anti-PD-L2 antibody treatment.

Typically, the method of the present invention comprises a) quantifying the level of the soluble CD27 in the biological sample; b) implementing a classification algorithm on data comprising the quantified of sCD27 levels so as to obtain an algorithm output; c) determining the probability that the subject will achieve or not a response to an anti-PD-1, anti-PD-L1 or anti-PD-L2 antibody from the algorithm output of step b).

In some embodiments, the method according to the invention wherein the algorithm is selected from Linear Discriminant Analysis (LDA), Topological Data Analysis (TDA), Neural Networks, Support Vector Machine (SVM) algorithm and Random Forests algorithm (RF).selected from Linear Discriminant Analysis (LDA), Topological Data Analysis (TDA), Neural Networks, Support Vector Machine (SVM) algorithm and Random Forests algorithm (RF).

In some embodiments, the method of the invention comprises the step of determining the subject response using a classification algorithm. As used herein, the term "classification algorithm" has its general meaning in the art and refers to classification and regression tree methods and multivariate classification well known in the art such as described in US 8,126,690; WO2008/156617. As used herein, the term "support vector machine (SVM)" is a universal learning machine useful for pattern recognition, whose decision surface is parameterized by a set of support vectors and a set of corresponding weights, refers to a method of not separately processing, but simultaneously processing a plurality of variables. Thus, the support vector machine is useful as a statistical tool for classification. The support vector machine non-linearly maps its n-dimensional input space into a high dimensional feature space, and presents an optimal interface (optimal parting plane) between features. The support vector machine comprises two phases: a training phase and a testing phase. In the training phase, support vectors are produced, while estimation is performed according to a specific rule in the testing phase.In general, SVMs provide a model for use in classifying each of n subjects to two or more disease categories based on one k-dimensional vector (called a k-tuple) of biomarker measurements per subject. An SVM first transforms the k-tuples using a kernel function into a space of equal or higher dimension. The kernel function projects the data into a space where the categories can be better separated using hyperplanes than would be possible in the original data space. To determine the hyperplanes with which to discriminate between categories, a set of support vectors, which lie closest to the boundary between the disease categories, may be chosen. A hyperplane is then selected by known SVM techniques such that the distance between the support vectors and the hyperplane is maximal within the bounds of a cost function that penalizes incorrect predictions. This hyperplane is the one which optimally separates the data in terms of prediction (Vapnik, 1998 Statistical Learning Theory. New York: Wiley). Any new observation is then classified as belonging to any one of the categories of interest, based where the observation lies in relation to the hyperplane. When more than two categories are considered, the process is carried out pairwise for all of the categories and those results combined to create a rule to discriminate between all the categories. As used herein, the term "Random Forests algorithm" or "RF" has its general meaning in the art and refers to classification algorithm such as described in US 8,126,690; WO2008/156617. Random Forest is a decision-tree-based classifier that is constructed using an algorithm originally developed by Leo Breiman (Breiman L, "Random forests," Machine Learning 2001, 45:5-32). The classifier uses a large number of individual decision trees and decides the class by choosing the mode of the classes as determined by the individual trees. The individual trees are constructed using the following algorithm: (1) Assume that the number of cases in the training set is N, and that the number of variables in the classifier is M; (2) Select the number of input variables that will be used to determine the decision at a node of the tree; this number, m should be much less than M; (3) Choose a training set by choosing N samples from the training set with replacement; (4) For each node of the tree randomly select m of the M variables on which to base the decision at that node; (5) Calculate the best split based on these m variables in the training set. In some embodiments, the score is generated by a computer program.

The algorithm of the present invention can be performed by one or more programmable processors executing one or more computer programs to perform functions by operating on input data and generating output. The algorithm can also be performed by, and apparatus can also be implemented as, special purpose logic circuitry, e.g., an FPGA (field programmable gate array) or an ASIC (application-specific integrated circuit). Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device. Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry. To provide for interaction with a user, embodiments of the invention can be implemented on a computer having a display device, e.g., in non-limiting examples, a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. Accordingly, in some embodiments, the algorithm can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the invention, or any combination of one or more such back-end, middleware, or front-end components. The components of the system can be interconnected by any form or medium of digital data communication, e.g., a communication network. Examples of communication networks include a local area network ("LAN") and a wide area network ("WAN"), e.g., the Internet. The computing system can include clients and servers. A client and server are generally remote from each other and typically interact through a communication network. The relationship of client and server arises by virtue of computer programs running on the respective computers and having a client-server relationship to each other.

As used herein, the term "immune checkpoint inhibitor" refers to molecules that totally or partially reduce, inhibit, interfere with or modulate one or more immune checkpoint proteins.

As used herein, the term "immune checkpoint protein" has its general meaning in the art and refers to a molecule that is expressed by T cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al. 2011. Nature 480:480- 489). Examples of stimulatory checkpoint include CD27 CD28 CD40, CD122, CD137, OX40, GITR, and ICOS. Examples of inhibitory checkpoint molecules include A2AR, B7-H3, B7-H4, BTLA, CTLA-4, CD277, IDO, KIR, PD-1, LAG-3, TIM-3 and VISTA. The Adenosine A2A receptor (A2AR) is regarded as an important checkpoint in cancer therapy because adenosine in the immune microenvironment, leading to the activation of the A2a receptor, is negative immune feedback loop and the tumor microenvironment has relatively high concentrations of adenosine. B7-H3, also called CD276, was originally understood to be a co-stimulatory molecule but is now regarded as co-inhibitory. B7-H4, also called VTCN1, is expressed by tumor cells and tumor-associated macrophages and plays a role in tumour escape. B and T Lymphocyte Attenuator (BTLA) and also called CD272, has HVEM (Herpesvirus Entry Mediator) as its ligand. Surface expression of BTLA is gradually downregulated during differentiation of human CD8+ T cells from the naive to effector cell phenotype, however tumor-specific human CD8+ T cells express high levels of BTLA. CTLA-4, Cytotoxic T-Lymphocyte-Associated protein 4 and also called CD152. Expression of CTLA-4 on Treg cells serves to control T cell proliferation. IDO, Indoleamine 2,3-dioxygenase, is a tryptophan catabolic enzyme. A related immune-inhibitory enzymes. Another important molecule is TDO, tryptophan 2,3-dioxygenase. IDO is known to suppress T and NK cells, generate and activate Tregs and myeloid-derived suppressor cells, and promote tumour angiogenesis. KIR, Killer-cell Immunoglobulin-like Receptor, is a receptor for MHC Class I molecules on Natural Killer cells. LAG3, Lymphocyte Activation Gene-3, works to suppress an immune response by action to Tregs as well as direct effects on CD8+ T cells. PD-1, Programmed Death 1 (PD-1) receptor, has two ligands, PD-L1 and PD-L2. This checkpoint is the target of Merck & Co.'s melanoma drug Keytruda, which gained FDA approval in September 2014. An advantage of targeting PD-1 is that it can restore immune function in the tumor microenvironment. TIM-3, short for T-cell Immunoglobulin domain and Mucin domain 3, expresses on activated human CD4+ T cells and regulates Th1 and Th17 cytokines. TIM-3 acts as a negative regulator of Th1/Tc1 function by triggering cell death upon interaction with its ligand, galectin-9. VISTA, Short for V-domain Ig suppressor of T cell activation, VISTA is primarily expressed on hematopoietic cells so that consistent expression of VISTA on leukocytes within tumors may allow VISTA blockade to be effective across a broad range of solid tumors. Tumor cells often take advantage of these checkpoints to escape detection by the immune system. Thus, inhibiting a checkpoint protein on the immune system may enhance the anti-tumor T-cell response.

In a particular embodiment, the immune checkpoint inhibitor is an antibody.

In a particular embodiment, the immune checkpoint inhibitor is an anti-PD-1 antibody such as described in WO2011082400, WO2006121168, WO2015035606, WO2004056875, WO2010036959, WO2009114335, WO2010089411, WO2008156712, WO2011110621, WO2014055648 and WO2014194302. Examples of anti-PD-1 antibodies which are commercialized: Nivolumab (Opdivo^{®}, BMS), Pembrolizumab (also called Lambrolizumab, KEYTRUDA^{®} or MK-3475, MERCK).

In some embodiments, the immune checkpoint inhibitor is an anti-PD-L1 antibody such as described in WO2013079174, WO2010077634, WO2004004771, WO2014195852, WO2010036959, WO2011066389, WO2007005874, WO2015048520, US8617546 and WO2014055897. Examples of anti-PD-L1 antibodies which are on clinical trial: Atezolizumab (MPDL3280A, Genentech/Roche), Durvalumab (AZD9291, AstraZeneca), Avelumab (also known as MSB0010718C, Merck) and BMS-936559 (BMS).

In some embodiments, the immune checkpoint inhibitor is an anti-PD-L2 antibody such as described in US7709214, US7432059 and US8552154.

### Method for treating a subject identified as responder to an immune checkpoint inhibitor treatment

In a second aspect, the invention relates to a method for treating kidney cancer in a subject identified as a responder to an immune checkpoint inhibitor treatment selected from the group consisting of anti-PD-1, anti-PD-L1 or anti-PD-L2 antibody, which results in i) quantifying the level of soluble CD27 in a biological sample obtained from the subject before being treated with an anti-PD-1, anti-PD-L1 or anti-PD-L2 antibody; ii) providing an evaluation based on the quantity of the soluble CD27 in the biological sample of said subject; and iii) treating said subject with an anti-PD-1, anti-PD-L1 or anti-PD-L2 antibody if said subject is identified as responder to said treatment at step ii).

Also described but not part of the invention is a method of treating renal cancer in a subject identified as a responder with an anti-PD-1, anti-PD-L1 or anti-PD-L2 antibody comprising the steps of i) quantifying the level of soluble CD27 in a biological sample obtained from the subject treated with an anti-PD-1, anti-PD-L1 or anti-PD-L2 antibody, ii) comparing the level of soluble CD27 quantified at step i) with its corresponding predetermined reference values, iii) concluding that the subject will not respond to the treatment when the level of soluble CD27 is higher than its corresponding predetermined reference value or concluding that the subject will respond to the treatment when the level of soluble CD27 is lower than its corresponding predetermined reference value and iv) treating said subject with an anti-PD-1, anti-PD-L1 or anti-PD-L2 antibody.

As used herein, the terms "treating" or "treatment" refer to both prophylactic or preventive treatment as well as curative or disease modifying treatment, including treatment of subject at risk of contracting the disease or suspected to have contracted the disease as well as subject who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, the term "subject" denotes a mammal, such as a rodent, a feline, a canine, and a primate. Particularly, the subject according to the invention is a human. More particularly, the subject according to the invention has or is susceptible to have renal cell cancer (RCC).

As used herein, the term "cancer" refers to a malignant growth or tumour resulting from an uncontrolled division of cells as defined above.

As used herein, the term "immune checkpoint inhibitor" refers to molecules that totally or partially reduce, inhibit, interfere with or modulate one or more immune checkpoint proteins. Such immune checkpoint inhibitors are defined above.

In a particular embodiment, the method according to the invention, wherein at least two immune checkpoint inhibitors selected from the group consisting of anti-PD-1, anti-PD-L1 or anti-PD-L2 antibody are used as a combined preparation for treating the subject identified as responder to an immune checkpoint inhibitor treatment.

In a further embodiment, the method according to the invention, wherein an anti-PD-1 antibody and an anti-CTLA-4 antibody are used as a combined preparation for treating the subject identified as responder to an immune checkpoint inhibitor treatment.

In a particular embodiment, the method according to the invention, wherein an anti-PD-L1 antibody and an anti-CTLA-4 antibody are used as a combined preparation for treating the subject identified as responder to an immune checkpoint inhibitor treatment.

In a particular embodiment, i) an immune checkpoint inhibitor selected from the group consisting of anti-PD-1, anti-PD-L1 or anti-PD-L2 antibody and ii) an anti-angiogenesis compound are used as a combined preparation for treating the subject identified as responder to an immune checkpoint inhibitor treatment.

As used herein, the term "angiogenesis" refers to a physiological process involving the growth of new blood vessels from preexisting vessels. Angiogenesis is a combinatorial process that is regulated by a balance between pro- and anti-angiogenic molecules. Angiogenic stimuli (e.g. hypoxia or inflammatory cytokines) result in the induced expression and release of angiogenic growth factors such as vascular endothelial growth factor (VEGF) or fibroblast growth factor (FGF).

As used herein, the term "anti-angiogenesis" refers to any molecule which can inhibit (anti- angiogenesis) the creation of new blood vessels. Typically, anti-angiogenesis compound is well known in the art and refers to the following compounds but not limited to bevacizumab (Avastin, anti-VEGF), itraconazole (anti-VGFR), carboxyamidotriazole, TNP-470 (an analog of fumagillin), CM101, IFN-α, IL-12, platelet factor-4, suramin, SU5416, thrombospondin, VEGFR antagonists, angiostatic steroids + heparin, Cartilage-Derived Angiogenesis Inhibitory Factor, matrix metalloproteinase inhibitors, angiostatin, endostatin, 2-methoxyestradiol, tecogalan, tetrathiomolybdate, thalidomide, thrombospondin, prolactin, αVβ3 inhibitors, linomide, ramucirumab, tasquinimod, ranibizumab, sorafenib (Nexavar), sunitinib (Sutent), pazopanib (Votrient), everolimus (Afinitor), cabozantinib.

In a particular embodiment, the method according to the invention, wherein, i) the immune checkpoint inhibitor selected from the group consisting of anti-PD-1, anti-PD-L1 or anti-PD-L2 antibody and ii) a chemotherapeutic agent or a radiation therapy are used as combined preparation for treating the subject identified as responder to an immune checkpoint inhibitor treatment.

As used herein, the terms "combined treatment", "combined therapy" or "therapy combination" refer to a treatment that uses more than one medication. The combined therapy may be dual therapy or bi-therapy.

In a particular embodiment, at least two immune checkpoint inhibitors selected from the group consisting of anti-PD-1, anti-PD-L1 or anti-PD-L2 antibody as a combined preparation according to the invention for simultaneous, separate or sequential use in the method for treating a cancer.

In a particular embodiment, i) an immune checkpoint inhibitor selected from the group consisting of anti-PD-1, anti-PD-L1 or anti-PD-L2 antibody and ii) an anti-angiogenesis compound as a combined preparation according to the invention for simultaneous, separate or sequential use in the method for treating a cancer.

In a particular embodiment, i) an immune checkpoint inhibitor selected from the group consisting of anti-PD-1, anti-PD-L1 or anti-PD-L2 antibody and a chemotherapeutic agent or a radiotherapeutics agent as a combined preparation according to the invention for simultaneous, separate or sequential use in the method for treating a cancer.

As used herein, the term "simultaneous use" refers to an administration of 2 active ingredients by the same route and at the same time or at substantially the same time. The term "separate use" refers to an administration of 2 active ingredients at the same time or at substantially the same time by different routes. The term "sequential use" refers to an administration of 2 active ingredients at different times, the administration route being identical or different.

As used herein, the term "chemotherapeutic agent" refers to chemical compounds that are effective in inhibiting tumor growth. Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaorarnide and trimethylolomelamine; acetogenins (especially bullatacin and bullatacinone); a carnptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33: 183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalarnycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophospharnide glycoside; aminolevulinic acid; amsacrine; bestrabucil; bisantrene; edatraxate; defo famine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin; podophyllinic acid; 2-ethylhydrazide; procarbazine; PSK^{®}; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.].) and doxetaxel (TAXOTERE^{®}, Rhone-Poulenc Rorer, Antony, France); chlorambucil; gemcitabine; 6-thioguanine; mercaptopurine; methotrexate; platinum analogs such as cisp latin and carbop latin; vinblastine; platinum; etoposide (VP- 16); ifosfamide; mitomycin C; mitoxantrone; vincristine; vinorelbine; navelbine; novantrone; teniposide; daunomycin; aminopterin; xeloda; ibandronate; CPT-1 1 ; topoisomerase inhibitor RFS 2000; difluoromethylornithine (DMFO); retinoic acid; capecitabine; and pharmaceutically acceptable salts, acids or derivatives of any of the above. Also included in this definition are antihormonal agents that act to regulate or inhibit honnone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

As used herein, the term "radiation therapy" has its general meaning in the art and refers the treatment of cancer with ionizing radiation. Ionizing radiation deposits energy that injures or destroys cells in the area being treated (the target tissue) by damaging their genetic material, making it impossible for these cells to continue to grow. One type of radiation therapy commonly used involves photons, e.g. X-rays. Depending on the amount of energy they possess, the rays can be used to destroy cancer cells on the surface of or deeper in the body. The higher the energy of the x-ray beam, the deeper the x-rays can go into the target tissue. Linear accelerators and betatrons produce x-rays of increasingly greater energy. The use of machines to focus radiation (such as x-rays) on a cancer site is called external beam radiation therapy. Gamma rays are another form of photons used in radiation therapy. Gamma rays are produced spontaneously as certain elements (such as radium, uranium, and cobalt 60) release radiation as they decompose, or decay. In some embodiments, the radiation therapy is external radiation therapy. Examples of external radiation therapy include, but are not limited to, conventional external beam radiation therapy; three-dimensional conformal radiation therapy (3D-CRT), which delivers shaped beams to closely fit the shape of a tumor from different directions; intensity modulated radiation therapy (IMRT), e.g., helical tomotherapy, which shapes the radiation beams to closely fit the shape of a tumor and also alters the radiation dose according to the shape of the tumor; conformal proton beam radiation therapy; image-guided radiation therapy (IGRT), which combines scanning and radiation technologies to provide real time images of a tumor to guide the radiation treatment; intraoperative radiation therapy (IORT), which delivers radiation directly to a tumor during surgery; stereotactic radiosurgery, which delivers a large, precise radiation dose to a small tumor area in a single session; hyperfractionated radiation therapy, e.g., continuous hyperfractionated accelerated radiation therapy (CHART), in which more than one treatment (fraction) of radiation therapy are given to a subject per day; and hypofractionated radiation therapy, in which larger doses of radiation therapy per fraction is given but fewer fractions.

In some embodiments, the method of the present invention is particularly suitable in the context of a hypo fractionated radiation therapy. As used herein the term "hypo fractionated radiation therapy" has its general meaning in the art and refers to radiation therapy in which the total dose of radiation is divided into large doses and treatments are given less than once a day.

### Kits or devices of the present invention

A third aspect of the present invention relates to use of a kit or device for performing the method of the present invention, comprising means for determining the level of the soluble CD27 in a biological sample.

In some embodiments, the kit or device comprises at least one binding partner (e.g. antibody or aptamer) specific for the soluble CD27 (immobilized or not on a solid support as described above). In some embodiments, the kit or device can include a second binding partner (e.g. antibody or aptamer) of the present invention which produces a detectable signal. Examples of kits include but are not limited to ELISA assay kits, and kits comprising test strips and dipsticks.

In some embodiments, the kit or device of the present invention further comprises a microprocessor to implement an algorithm on data comprising the level of soluble CD27 in the sample so as to determine the probability of responding to an immune checkpoint inhibitor. In some embodiments, the kit or device of the present invention further comprises a visual display and/or audible signal that indicates the probability determined by the microprocessor.

In some embodiments, the kit or device of the present invention comprises:
- a mass spectrometer;
- a receptacle into which the biological sample is placed, and which is connectable to the mass spectrometer so that the mass spectrometer can quantify the level of soluble CD27 in the sample;
- a microprocessor to implement an algorithm on data comprising the levels of soluble CD27 in the sample so as to determine the probability of responding to an immune checkpoint inhibitor;
- a visual display and/or audible signal that indicates the probability determined by the microprocessor.

The disclosure will be further illustrated by the following figures and examples. However, these examples and figures should not be interpreted as according to the invention and in any way as limiting the scope of the present disclosure.

### FIGURES:

**Figure 1****: Plasma levels of CD27 and Tim-3 are associated with the survival of patients with renal cell cancer patients treated with anti-PD-1 / PD-L1.** Plasma concentrations of soluble CD27 **(A.)** and soluble Tim-3 **(B.)** were measured prior to anti-PD-1 / PD-L1 therapy in 27 patients with metastatic renal cell carcinoma. Patients were divided into two groups selected depending on their plasma CD27 concentrations (above or below the median). Patient survival was determined from the start of treatment. A Log Rank test was performed to compare the survival of the 2 groups of patients.

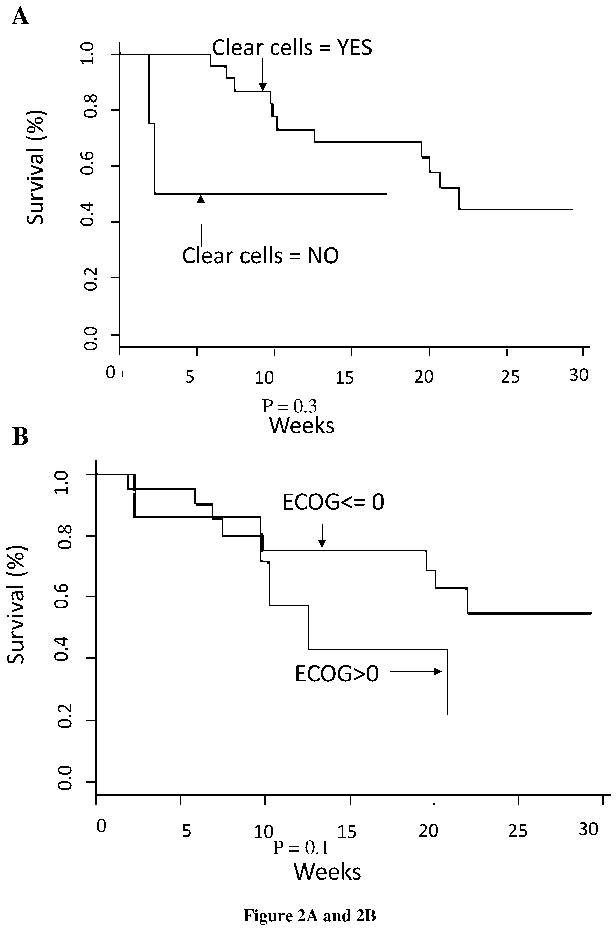

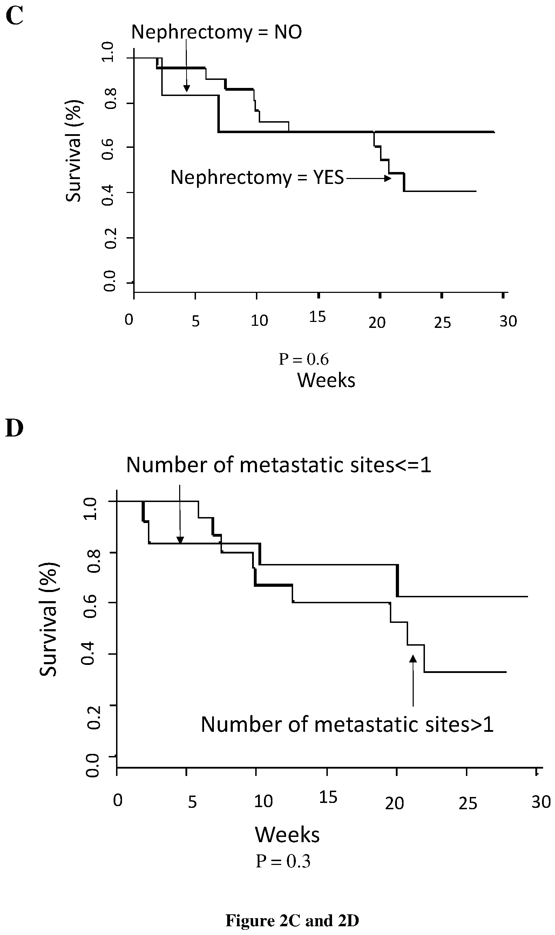

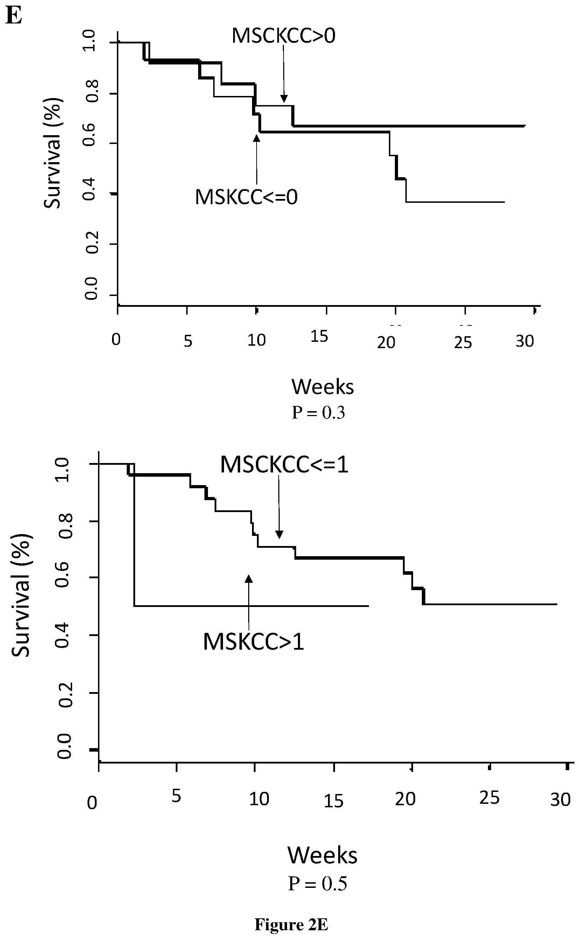

**Figure 2****: Lack of correlation between prognostic clinical parameters associated** with **renal cell cancer and survival of patients treated with anti-PD-1 / PD-L1.**
   Different clinical parameters **(A.** histological type of tumor, **B.** ECOG, **C.** anterior nephrectomy, **D.** number of metastatic sites, **E.** MSKCC criteria) whose prognostic value is recognized have been correlated with the survival of patients treated with anti-PD-1 / PD-L1.

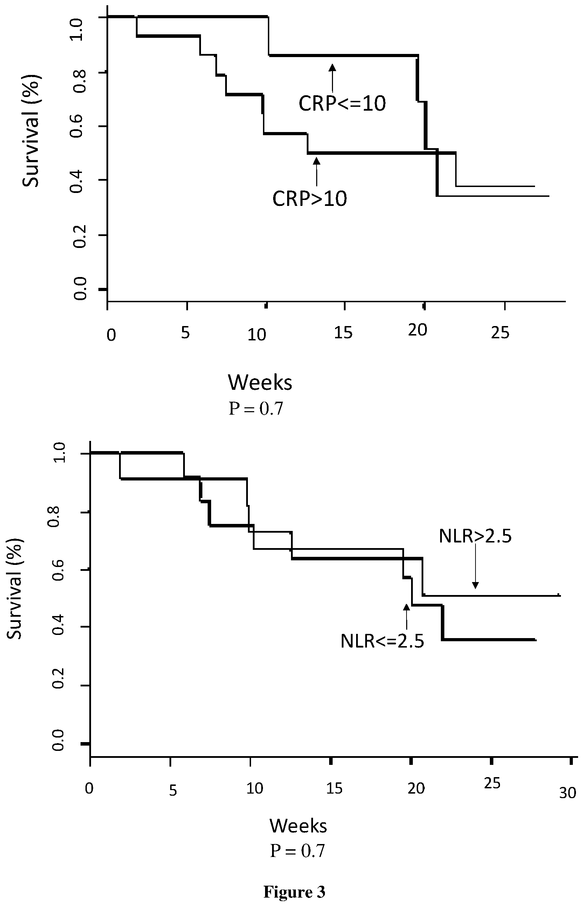

**Figure 3****: Lack of correlation between biological parameters linked to inflammation and survival of patients with renal cell cancer treated with anti-PD-1 / PD-L1.** Two parameters related to inflammation (CRP, Neutrophil / Lymphocyte Ratio) were measured before treatment with anti-PD-1 / PD-L1 in the same series of patients with metastatic renal cell cancer. The variables were dichotomized according to the threshold defined to consider the value as pathological. Similar results were observed when median values were used to divide patients into 2 groups or with a threshold for CRP at 5 mg / L.

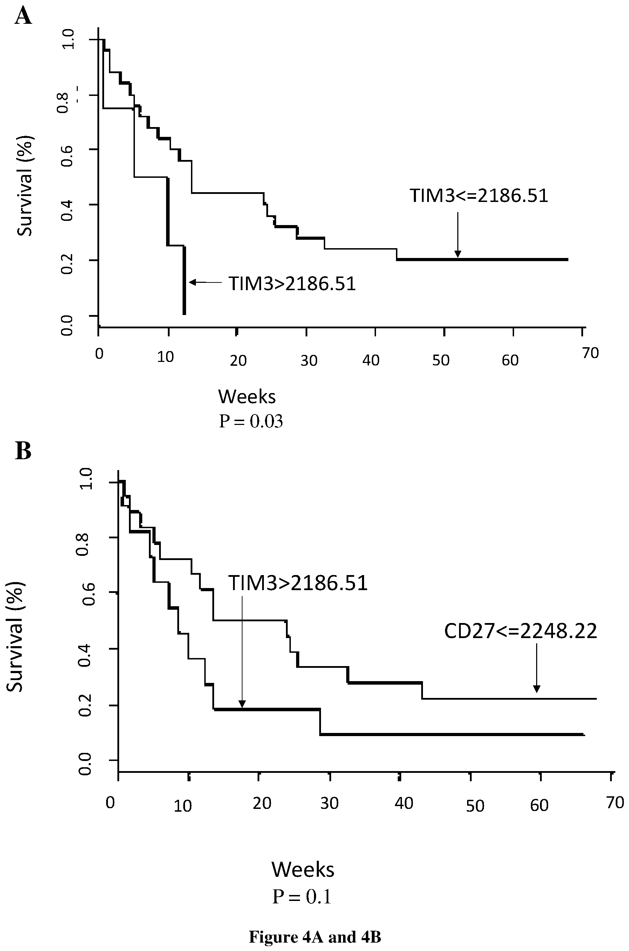

**Figure 4****: Analysis of correlation between plasma levels of CD27 and Tim-3 and survival of patients with renal cell cancer treated with sunitinib (anti-angiogenic molecule).** Plasma concentrations of **A.** soluble CD27 and **B.** soluble Tim-3 were measured before treatment with an anti-angiogenic molecule, sunitinib, in 27 patients with metastatic renal cell cancer. The patients were divided into two groups selected according to their values above or below the median of the plasma concentrations determined previously (see FIG. 1). Patient survival was determined from the start of treatment. A Log Rank test was performed to compare the survival of the 2 groups of patients.

### EXAMPLE:

### Material & Methods

### Patients: We selected in this project two cohorts of patients:

- A colcheckpoint cohort set up at the Georges Pompidou European Hospital (CPP: 2015-08-04-MS2) including since 2016 all the patients treated by immunotherapy (Anti-PD-1 / PD-L1) since 2016 and for whom blood and tissue samples are routinely performed after collection of patient consent. 27 renal cell cancer patients derived from this cohort were included in this study. 22 of these patients had clear cell kidney cancer.
- The other set of specimens came from the Preinsut clinical study, where renal cell cancer patients received two cycles of sunitinib before nephrectomy [8]. 27 patients derived from this cohort were also included in this study

### Plasma

The plasmas of these two cohorts of patients were obtained from the biological resources platform of the Georges Pompidou European Hospital (Claudia de Toma)

### Clinical and biological data

Different clinical data (Histological type of the tumor, ECOG status, number of metastatic sites, MSKCC criteria, patient survival) and biological data (CRP, NLR) were collected. These latter parameters (CRP, NLR...) were selected, because they are most often associated with the inflammatory status of the patients.

### Measurement of soluble receptors

The Procartaplex kit (Thermofischer) was used to carry out a panel of soluble inhibitor or activator receptor (BTLA, GITR, HVEM, IDO, LAG-3, PD-1, PD-L1, PD-L2, Tim-3, CD28, CD80, 4-1BB, CD27, and CTLA-4)

### Statistical analysis

Log Rank (Kaplan Meier) or Cox tests were used to correlate the different parameters with patient survival.

The different variables were analyzed either qualitatively with a threshold at the median for dichotomizing them or quantitatively (Cox model)

### Results

### 1) Correlation between plasma concentrations of soluble CD27 and soluble Tim-3 and survival of renal cancer patients treated with anti-PD-1/PD-L1.

In the cohort of colcheckpoint patients treated with immunotherapy, only pre-therapeutic plasma concentrations of 2 parameters (Tim-3 and CD27) measured before the start of treatment were correlated with patient survival (Fig 1 A and B) in a statistically significant manner.

For the CD27 marker, this correlation was found whatever the statistical test used : Log Rank (qualitative variable) (p = 0.005) (Fig 1A), or Cox model (Quantitative variable) (Table 1) (p = 0.04).

**Table 1: Correlation between plasma concentrations of soluble CD27 and soluble Tim-3 and survival determined by a Cox model.**

| | RR | IC inf | IC sup | p-value |
|---|---|---|---|---|
| CD27 | 1 | 1 | 1.001 | 0.041 |
| TIM3 | 1 | 1 | 1.001 | 0.1 |

### 2) No correlation between clinical and conventional biological prognostic criteria and response to anti-PD-1/PD-L1 immunotherapy.

Interestingly, in this same series of patients, none of the clinical criteria (histological type, ECOG, anterior nephrectomy, number of metastatic sites, MSKCC criteria) (FIG. 2A-E) or biological criteria (CRP, neutrophil / lymphocyte ratio (NLR)) (FIG. 3) was associated with the survival of these immunotherapy-treated patients.

Different clinical parameters (histological type of tumor, ECOG, anterior nephrectomy, number of metastatic sites, MSKCC criteria) whose prognostic value is recognized have been correlated with the survival of patients treated with anti-PD-1 / PD-L1.

### 3) Plasma concentrations of CD27 are not correlated with the survival of patients treated with antiangiogenic drugs.

In order to verify if these 2 parameters are predictive of the response to immunotherapy or rather prognostic markers of the clinical outcome of patients with metastatic renal cell cancer, we measured them in a cohort of patients treated with an antiangiogenic agent, the sunitinib. We found a correlation between pre-treatment plasma concentrations of Tim-3 and patient survival (p = 0.03) (Fig 4A), suggesting that this marker is more likely to be a prognostic factor in metastatic renal cell cancer and thus non really specific of the prediction of the response to immunotherapy.
In contrast, plasma CD27 concentrations were not correlated with patient survival (Fig 4B) suggesting that this marker is more related and predictive for anti-PD-1 / PD-L1 response.

### 4) The CD27 marker is not correlated with clinical prognostic criteria to classify metastatic renal cell cancers

We have shown that plasma concentrations of soluble CD27 do not correlate with the different clinical parameters used to classify patients with metastatic renal cell cancer, because of their prognostic role (ECOG, previous nephrectomy, number of metastatic sites, MSKCC criteria, etc.) (Table 2).

**Table 2: Correlation between Plasma soluble CD27 Concentrations and prognostic clinical criteria in Patients with Metastatic Renal cell Cancer treated by and Anti-PD-1 / PD-L1.**

| | Histological types : clear cell carcinoma | | |
|---|---|---|---|
| | Yes | NO | p-value |
| CD27 | 2309.6 [1318.7 ; 3597.0] | 1823.8 [892.0 ; 4940.1] | 0.76 |

| | ECOG | | |
|---|---|---|---|
| | 0 | 1/2 | p-value |
| CD27 | 2094.4 [1090.9 ; 3710.5] | 2502.6 [1465.4 ; 3338.4] | 0.99 |

| | Néphrectomy | | |
|---|---|---|---|
| | Yes | NO | p-value |
| CD27 | 2371.0 [1308.1 ; 3764.8] | 1498.8 [1058.2 ; 2264.7] | 0.29 |

| | Number metastatic sites | | |
|---|---|---|---|
| | 1 | 2/3 | p-value |
| CD27 | 1450.8 [601.8 ; 2764.3] | 2914.3 [1534.6 ; 3688.8] | 0.17 |

| | MSKCC Criteria | | |
|---|---|---|---|
| | 0 | >=1 | p-value |
| CD27 | 3231.9 [1418.5 ; 3943.7] | 1498.8 [864.5 ; 2403.9] | 0.059 |

| | MSKCC criteria | | |
|---|---|---|---|
| | 0/1 | >=2 | p-value |
| CD27 | 2094.4 [1267.3 ; 3565.4] | 1317.9 [725.6 ; 1910.2] | 0.39 |

### Conclusion

We have identified a soluble marker, CD27, present in the plasma of patients with renal cell cancer whose pre-treatment concentrations predict the response to anti-PD-1 / PD-L1.

This marker appears more as a predictive marker of response to anti-PD1 / PD-L1 treatment, than as a prognostic marker. Indeed, it is not associated with better survival in patients with metastatic renal cell cancer treated with an antiangiogenic agent.

This marker is not correlated with conventional clinical markers of severity that classify patients with metastatic renal cancer.

### REFERENCES:

Throughout this application, various references describe the state of the art to which this disclosure pertains.

## Claims

1. A method for determining whether a subject suffering from kidney cancer will achieve a response with an anti-PD-1, anti-PD-L1 or anti-PD-L2 antibody comprising the steps of i) quantifying the level of soluble CD27 in a biological sample obtained from the subject treated with an anti-PD-1, anti-PD-L1 or anti-PD-L2 antibody, ii) comparing the level of soluble CD27 quantified at step i) with its corresponding predetermined reference value and iii) concluding that the subject will not respond to the treatment when the level of soluble CD27 is higher than its corresponding predetermined reference value or concluding that the subject will respond to the treatment when the level of soluble CD27 is lower than its corresponding predetermined reference value.

2. An anti-PD-1, anti-PD-L1 or anti-PD-L2 antibody for use in the treatment of kidney cancer in a subject identified as a responder to an anti-PD-1, anti-PD-L1 or anti-PD-L2 antibody treatment, comprising the steps of i) quantifying the level of soluble CD27 in a biological sample obtained from the subject before being treated with ann anti-PD-1, anti-PD-L1 or anti-PD-L2 antibody; ii) providing an evaluation based on the quantity of the soluble CD27 in the biological sample of said subject; and iii) treating said subject with the anti-PD-1, anti-PD-L1 or anti-PD-L2 antibody if said subject is identified as responder to an said treatment at step ii).

3. The method of claim 1, wherein the biological sample is plasma sample.

4. The method of claim 1, further comprises a step of classification of subject by an algorithm for determining whether said subject will achieve a response to an anti-PD-1, anti-PD-L1 or anti-PD-L2 antibody treatment.

5. The method of claim 4 wherein the algorithm is selected from Linear Discriminant Analysis (LDA), Topological Data Analysis (TDA), Neural Networks, Support Vector Machine (SVM) algorithm and Random Forests algorithm (RF).

6. The anti-PD-1, anti-PD-L1 or anti-PD-L2 antibody for use according to claim 2, wherein at least two immune checkpoint inhibitors are used as a combined preparation for treating the subject identified as responder to an anti-PD-1, anti-PD-L1 or anti-PD-L2 antibody treatment.

7. The anti-PD-1, anti-PD-L1 or anti-PD-L2 antibody for use according to claim 2, wherein, i) the anti-PD-1, anti-PD-L1 or anti-PD-L2 antibody and ii) a chemotherapeutic agent or a radiotherapeutics agent are used as combined preparation for treating the subject identified as responder to an anti-PD-1, anti-PD-L1 or anti-PD-L2 antibody treatment.

8. The anti-PD-1, anti-PD-L1 or anti-PD-L2 antibody for use according to claim 2, i) an anti-PD-1, anti-PD-L1 or anti-PD-L2 antibody and ii) an anti-angiogenesis compound are used as a combined preparation for treating the subject identified as responder to an anti-PD-1, anti-PD-L1 or anti-PD-L2 antibody treatment.

9. Use of a kit or device for performing the method of claim 1, wherein the kit comprises means for determining the level of the soluble CD27 in a biological sample.

## Patentansprüche

1. Verfahren zum Bestimmen, ob ein an Nierenkrebs leidender Patient auf die Behandlung mit einem Anti-PD-1-, Anti-PD-L1- oder Anti-PD-L2-Antikörper anspricht, umfassend die Schritte zum i) Quantifizieren des Spiegels an löslichem CD27 in einer biologischen Probe, die von dem mit einem Anti-PD-1-, Anti-PD-L1- oder Anti-PD-L2-Antikörper behandelten Patienten gewonnen wurde, ii) Vergleichen des in Schritt i) quantifizierten Spiegels an löslichem CD27 mit seinem entsprechenden vorbestimmten Referenzwert und iii) Schlussfolgern, dass der Patient nicht auf die Behandlung anspricht, wenn der Spiegel an löslichem CD27 höher ist als sein entsprechender vorbestimmter Referenzwert, oder Schlussfolgern, dass der Patient auf die Behandlung anspricht, wenn der Spiegel an löslichem CD27 niedriger ist als sein entsprechender vorbestimmter Referenzwert.

2. Anti-PD-1-, Anti-PD-L1- oder Anti-PD-L2-Antikörper zur Verwendung bei der Behandlung von Nierenkrebs bei einem Patienten, der als auf eine Behandlung mit einem Anti-PD-1-, Anti-PD-L1- oder Anti-PD-L2-Antikörper ansprechend identifiziert wurde, umfassend die Schritte zum i) Quantifizieren des Spiegels an löslichem CD27 in einer biologischen Probe, die vom Patienten vor der Behandlung mit einem Anti-PD-1-, Anti-PD-L1- oder Anti-PD-L2-Antikörper gewonnen wurde; ii) Bereitstellen einer Bewertung basierend auf der Menge des löslichen CD27 in der biologischen Probe des Patienten; und iii) Behandeln des Patienten mit dem Anti-PD-1-, Anti-PD-L1- oder Anti-PD-L2-Antikörper, wenn der Patient in Schritt ii) als auf die Behandlung ansprechend identifiziert wurde.

3. Verfahren nach Anspruch 1, wobei die biologische Probe eine Plasmaprobe ist.

4. Verfahren nach Anspruch 1, weiter umfassend einen Schritt zur Klassifizierung des Patienten durch einen Algorithmus zum Bestimmen, ob der Patient auf eine Behandlung mit einem Anti-PD-1-, Anti-PD-L1- oder Anti-PD-L2-Antikörper anspricht.

5. Verfahren nach Anspruch 4, wobei der Algorithmus aus der linearen Diskriminanzanalyse (LDA), topologischen Datenanalyse (TDA), neuronalen Netzwerken, Support Vector Machine- (SVM)-Algorithmus und Random Forests-Algorithmus (RF) ausgewählt wird.

6. Anti-PD-1-, Anti-PD-L1- oder Anti-PD-L2-Antikörper zur Verwendung nach Anspruch 2, wobei mindestens zwei Immun-Checkpoint-Inhibitoren als Kombinationspräparat zur Behandlung des Patienten verwendet werden, der als auf eine Anti-PD-1-, Anti-PD-L1- oder Anti-PD-L2-Antikörperbehandlung ansprechend identifiziert wurde.

7. Anti-PD-1-, Anti-PD-L1- oder Anti-PD-L2-Antikörper zur Verwendung nach Anspruch 2, wobei i) der Anti-PD-1-, Anti-PD-L1- oder Anti-PD-L2-Antikörper und ii) ein Chemotherapeutikum oder ein Radiotherapeutikum als Kombinationspräparat zum Behandeln des Patienten verwendet werden, der als auf eine Anti-PD-1-, Anti-PD-L1- oder Anti-PD-L2-Antikörperbehandlung ansprechend identifiziert wurde.

8. Anti-PD-1-, Anti-PD-L1- oder Anti-PD-L2-Antikörper zur Verwendung nach Anspruch 2, wobei i) ein Anti-PD-1-, Anti-PD-L1- oder Anti-PD-L2-Antikörper und ii) eine Anti-Angiogenese-Verbindung als Kombinationspräparat zum Behandeln des Patienten verwendet werden, der als auf eine Anti-PD-1-, Anti-PD-L1- oder Anti-PD-L2-Antikörperbehandlung ansprechend identifiziert wurde.

9. Verwendung eines Kits oder einer Vorrichtung zur Durchführung des Verfahrens nach Anspruch 1, wobei das Kit Mittel zum Bestimmen des Spiegels an löslichem CD27 in einer biologischen Probe umfasst.

## Revendications

1. Procédé pour déterminer si un sujet souffrant d'un cancer du rein obtiendra une réponse avec un anticorps anti-PD-1, anti-PD-L1 ou anti-PD-L2 comprenant les étapes consistant à i) quantifier le niveau de CD27 soluble dans un échantillon biologique obtenu du sujet traité avec un anticorps anti-PD-1, anti-PD-L1 ou anti-PD-L2, ii) comparer le niveau de CD27 soluble quantifié à l'étape i) à sa valeur de référence prédéterminée correspondante et iii) conclure que le sujet ne répondra pas au traitement lorsque le niveau de CD27 soluble est supérieur à sa valeur de référence prédéterminée correspondante ou conclure que le sujet répondra au traitement lorsque le niveau de CD27 soluble est inférieur à sa valeur de référence prédéterminée correspondante.

2. Anticorps anti-PD-1, anti-PD-L1 ou anti-PD-L2 destiné à être utilisé dans le traitement du cancer du rein chez un sujet identifié comme étant un sujet répondant à un traitement par anticorps anti-PD-1, anti-PD-L1 ou anti-PD-L2, comprenant les étapes consistant à i) quantifier le niveau de CD27 soluble dans un échantillon biologique obtenu du sujet avant d'être traité par un anticorps anti-PD-1, anti-PD-L1 ou anti-PD-L2 ; ii) fournir une évaluation sur la base de la quantité de CD27 soluble dans l'échantillon biologique dudit sujet ; et iii) traiter ledit sujet avec l'anticorps anti-PD-1, anti-PD-L1 ou anti-PD-L2 si ledit sujet est identifié comme étant un sujet répondant à un dit traitement à l'étape ii).

3. Procédé selon la revendication 1, dans lequel l'échantillon biologique est un échantillon de plasma.

4. Procédé selon la revendication 1, comprenant en outre une étape de classification du sujet par un algorithme pour déterminer si ledit sujet obtiendra une réponse à un traitement par anticorps anti-PD-1, anti-PD-L1 ou anti-PD-L2.

5. Procédé selon la revendication 4, dans lequel l'algorithme est choisi parmi une analyse discriminante linéaire (LDA), une analyse de données topologiques (TDA), des réseaux neuronaux, un algorithme de machine à vecteurs de support (SVM) et un algorithme de forêts d'arbres décisionnels (RF).

6. Anticorps anti-PD-1, anti-PD-L1 ou anti-PD-L2 destiné à être utilisé selon la revendication 2, dans lequel au moins deux inhibiteurs de point de contrôle immunitaire sont utilisés comme préparation combinée pour traiter le sujet identifié comme étant un sujet répondant à un traitement par anticorps anti-PD-1, anti-PD-L1 ou anti-PD-L2.

7. Anticorps anti-PD-1, anti-PD-L1 ou anti-PD-L2 destiné à être utilisé selon la revendication 2, dans lequel i) l'anticorps anti-PD-1, anti-PD-L1 ou anti-PD-L2 et ii) un agent chimiothérapeutique ou un agent radiothérapeutique sont utilisés comme préparation combinée pour traiter le sujet identifié comme étant un sujet répondant à un traitement par anticorps anti-PD-1, anti-PD-L1 ou anti-PD-L2.

8. Anticorps anti-PD-1, anti-PD-L1 ou anti-PD-L2 destiné à être utilisé selon la revendication 2, i) un anticorps anti-PD-1, anti-PD-L1 ou anti-PD-L2 et ii) un composé anti-angiogenèse sont utilisés comme préparation combinée pour traiter le sujet identifié comme étant un sujet répondant à un traitement par anticorps anti-PD-1, anti-PD-L1 ou anti-PD-L2.

9. Utilisation d'un kit ou d'un dispositif pour réaliser le procédé selon la revendication 1, dans lequel le kit comprend des moyens pour déterminer le niveau du CD27 soluble dans un échantillon biologique.
